Europäisches Patentamt

European Patent Office

Office européen des brevets

(19)

(11) Publication number: **0 075 355**
**B1**

(12) **EUROPEAN PATENT SPECIFICATION**

(45) Date of publication of patent specification: **28.08.85**

(21) Application number: **82201114.4**

(22) Date of filing: **09.09.82**

(51) Int. Cl.⁴: **C 07 C 69/65,** C 07 C 67/307, C 07 D 333/24

(54) The preparation of derivatives of dichloroacetic acid esters.

(30) Priority: **17.09.81 NL 8104287**

(43) Date of publication of application:
**30.03.83 Bulletin 83/13**

(45) Publication of the grant of the patent:
**28.08.85 Bulletin 85/35**

(84) Designated Contracting States:
**BE CH DE FR GB IT LI NL**

(56) References cited:

**BULLETIN DE LA SOCIETE CHIMIQUE DE FRANCE, 1959, pages 850-853, Paris (FR); M. JULIA et al.: "Sur quelques dérivés alpha-chloro et alpha, alpha-dichloroarylacétiques".**

(73) Proprietor: **STAMICARBON B.V.**
**Postbus 10**
**NL-6160 MC Geleen (NL)**

(72) Inventor: **Corvers, Antonius**
**Kelmonderstraat 33**
**NL-6191 RD Beek (NL)**
Inventor: **Mulders, Joannes M.C.A.**
**V.W. Goudoeverstraat 20**
**NL-6166 XC Geleen (NL)**

(74) Representative: **Hoogstraten, Willem Cornelis Roeland et al**
**OCTROOIBUREAU DSM Postbus 9**
**NL-6160 MA Geleen (NL)**

Courier Press, Leamington Spa, England.

## Description

The invention relates to a process for the preparation of derivatives of dichloroacetic acid esters by reaction of the corresponding glyoxylic acid esters with phosphoruspentachloride.

Such a reaction can be effected as known in the art by allowing powdery phosphoruspentachloride to act on the relative glyoxylic acid ester (see Bull. soc. chim. de France 1959, pages 850—853). Then the desired product is obtained with a yield in the order of 45—55% of the theoretically possible yield.

Now, the invention provides a process in the application of which the said reaction with phosphoruspentachloride can be effected with a substantially higher yield.

The process according to the invention for the preparation of derivatives of dichloroacetic acid ester, by reaction of the corresponding glyoxylic acid esters with phosphoruspentachloride, is characterized in that a glyoxylic acid ester having the general formula $R_1$—CO—$COOR_2$, wherein $R_1$ represents a phenyl, naphthyl, pyridyl, furyl or thienyl group which groups are optionally substituted with one or more substituents from the group consisting of Cl, $NO_2$, alkoxy with 1—8 C atoms and alkyl with 1—8 C atoms and $R_2$ an alkyl, cycloalkyl or aryl group, is reacted with the phosphoruspentachloride at a temperature of 35—150°C in the presence of a dispersant suitable for distributing the phosphoruspentachloride having a boiling point at a temperature of 35—150°C with formation of the corresponding dichloro derivative having the general formula $R_1$—$CCl_2$—$COOR_2$, wherein $R_1$ and $R_2$ have the said meanings.

In applying the process according to the invention the desired product can be recovered from the reaction mixture with sufficient purity, so that the product recovered can be used for further applications without having to be distilled.

The reaction with phosphoruspentachloride according to the invention is exothermic. Therefore, in order to maintain the desired temperature, the discharge of heat must be good. The desired reaction temperature is maintained preferably by using a distributor having a boiling point corresponding with the desired reaction temperature. The heat of reaction can then be carried off by evaporation of the dispersant, in which process the quantity of dispersant in the reaction mixture can be kept constant through application of reflux condensation.

The reaction temperature can be varied within the said limits. Preference is given to applying a temperature of between 70 and 100°C.

In applying the process according to the invention various dispersants suitable for distributing phosphoruspentachloride can be used, for instance alkanes or mixtures of alkanes, such as n-heptane and n-octane, cycloalkanes or mixtures of cycloalkanes, such as cyclopentane and cyclohexane, aromatics or mixtures of aromatics, such as benzene, toluene and xylenes and chlorinated hydrocarbons or mixtures of chlorinated hydrocarbons, such as chloroform, 1,2-dichloroethane and tetrachlorocarbon. Inorganic dispersants, too, will be eligible in principle. A suitable inorganic distributor is, for instance, phosphorusoxytrichloride.

In applying the process according to the invention various compounds may be started from in which the group $R_1$ in the said general formula represents a phenyl, a naphthyl, pyridyl, furyl or thienyl group, which groups may optionally be substituted with one or more substituents from the group consisting of Cl, $NO_2$, alkoxy with 1—8 C atoms and alkyl with 1—8 C atoms. As $R_2$ group in these starting compounds an alkyl group with 1—8 C atoms, a cycloalkyl group with 5—8 C atoms in the ring, a phenyl group or a naphthyl group, for instance, can be used. These starting compounds can be very effectively prepared in the way described in the non-prepublished European Patent Application 81201199.7 (EP—A—0053408).

The reaction mixture obtained according to the invention can, for instance, be worked up by distilling off the dispersant and the phosphorusoxytrichloride formed in the reaction, subsequently converting with water any phosphoruspentachloride present into phosphoric acid and hydrogen chloride, separating the organic layer formed from the aqueous layer, washing the organic layer with water and optionally purifying it by distillation. If in this working-up process the dispersant and the phosphorusoxytrichloride formed in the reaction process are not distilled off, an organic layer can be obtained which contains the dispersant and which can be used as such for further conversions. The reaction mixture can be worked up also by distilling off the dispersant and phosphorusoxytrichloride formed and incorporating the residue in an organic solvent in which phosphoruspentachloride precipitates such as, for instance, cyclohexane. After removal of the phosphoruspentachloride a solution will be left from which the product obtained can be recovered by distillation if so desired.

The process according to the invention, in the application of which products can be obtained that can be used in, for instance, the preparation of pesticides, will be further elucidated in the following examples.

### Example I

Into a flask (capacity = 1 liter), provided with a stirrer, reflux condenser, drop funnel and thermometer, 150 ml cyclohexane and 220 g phosphoruspentachloride are brought. The mixture is heated to about 90°C (boiling temperature), upon which 164,7 g phenylglyoxylic acid methylester is added in 40 minutes. After maintaining the reaction conditions for 1.5 hours, the gaschromatographic analysis shows that 99% of the quantity of glyoxylic acid ester is converted.

Subsequently the cyclohexane is distilled off, the remaining reaction mixture carefully poured out into one litre of water, and the mixture obtained extracted with ether. The organic layer is washed with a saturated NaHCO₃ solution to pH = 8 and dried with magnesiumsulphate. After evaporation of the ether, a residue (236.4 g) remains containing, according to gaschromatographic analysis, 92% of the desired chlorinated ester.

The yield is 93% of the theoretically possible yield. The boiling point of the product obtained is 95°C at a pressure of 133 Pa.

### Example II

In the way as indicated in Example I, 178 g phenylglyoxylic acid ethylester is converted into the dichloro derivative, but using 150 ml phosphorusoxytrichloride as dispersant.

After a reaction time of 1 hour, the phosphorusoxytrichloride is distilled off under reduced pressure, after which the product is worked up.

The yield is 96% of the theoretically possible yield. The product boils at 147°C at a pressure of $2.10^3$ Pa.

### Example III

Example I is repeated, using 476 g phosphoruspentachloride, 280 ml chloroform and 204.7 g phenylglyoxylic acid butylester.

After a reaction time of 5.5 hours, the desired dichloroester is obtained with a yield of 89% of the theoretically possible yield. The product boils at 99—100°C at a pressure of 27 Pa.

### Comparative Example

In equipment as described in Example I 20.6 g phenylglyoxylic acid butylester with 41.6 g powdery phosphoruspentachloride are heated, in the absence of a dispersant, to 80°C. After that the temperature of the reaction mixture rises to 170°C. Subsequently the reaction mixture is kept at 120°C (boiling temperature) for three hours more. The reaction mixture is then worked up in the way as described in Example I.

The desired ester is obtained with a yield of 55% of the theoretically possible yield.

### Example IV

In the way as described in Example I 5.6 g (p-methoxyphenyl)-glyoxylic acid ethylester in 5 ml cyclohexane is converted with 6.7 g phosphoruspentachloride into the dichloro derivative.

After 2.5 hours the desired dichloroester is obtained with a yield of 94% of the theoretically possible yield. The product boils at 105—107°C at a pressure of 13 Pa.

### Example V

In equipment as described in Example I 56 g (2-thienyl)-glyoxylic acid ethylester is added in drops to a boiling mixture of 65 ml cyclohexane and 90 g phosphoruspentachloride in 10 minutes. After maintaining the reaction conditions for 4 hours, the reaction mixture is cooled and worked up as follows:

Under reduced pressure the cyclohexane and the phosphorusoxytrichloride formed are distilled off, upon which the residue is incorporated in cyclohexane. The phosphoruspentachloride precipitated in this process is filtered off and subsequently pyridine is added in drops during stirring (binding of phosphoruspentachloride still dissolved) to the filtrate till no longer any precipitate is formed. After the precipitate has been filtered off and the solvent has been distilled off under reduced pressure, 71 g crude product is obtained containing 92% of the desired chlorinated ester. The yield is 90% of the theoretically possible yield. The product boils at 95°C at a pressure of 40 Pa.

### Claims

1. Process for the preparation of derivatives of dichloroacetic acid esters, by reaction of the corresponding glyoxylic acid esters with phosphoruspentachloride, characterized in that a glyoxylic acid ester having the general formula $R_1$—CO—COOR$_2$, wherein $R_1$ represents a phenyl, naphthyl, pyridyl, furyl or thienyl group, which groups are optionally substituted with one or more substituents from the group consisting of Cl, NO₂, alkoxy with 1—8 C atoms and alkyl with 1—8 C-atoms and $R_2$ an alkyl, cycloalkyl or aryl group, is reacted with the phosphoruspentachloride at a temperature of 35—150°C in the presence of a dispersant suitable for distributing the phosphoruspentachloride having a boiling point at a temperature of 35—150°C with formation of, the corresponding dichloro derivative having the general formula $R_1$—CCl$_2$—COOR$_2$, wherein $R_1$ and $R_2$ have the said meanings.

2. Process according to claim 1, characterized in that the reaction is effected at a temperature of 70—100°C.

### Patentansprüche

1. Verfahren zur Herstellung von Derivaten von Dichloressigsäureestern durch Umsetzung der entsprechenden Glyoxylsäureester mit Phosphorpentachlorid, dadurch gekennzeichnet, daß ein Glyoxylsäureester der allgemeinen Formel $R_1$—CO—COOR$_2$, worin R, eine Phenyl-Naphthyl-, Pyridyl-, Furyl- oder Thienylgruppe darstellt, welche Gruppen gegebenenfalls mit einem oder mehreren Substituenten aus der Gruppe bestehend aus Cl, NO₂, Alkoxy mit 1—8 Kohlenstoffatomen und Alkyl mit 1—8 Kohlenstoffatomen substituiert sind, mit dem Phosphorpentachlorid bei einer Temperatur von 35—150°C in Gegenwart eines für die Verteilung des Phosphorpentachlorids geeigneten Dispersionsmittels mit einem Siedepunkt bei einer Temperatur von 35—150°C unter Bildung des entsprechenden Dichlorderivates der Formel $R_1$—CCl$_2$—COOR$_2$, worin $R_1$ und $R_2$ obige Bedeutungen haben, umgesetzt wird.

2. Verfahren nach Anspruch 1, dadurch gekennzeichnet, dass die Reaktion bei einer Temperatur von 70—100°C stattfindet.

## Revendications

1. Procédé de préparation de dérivés d'esters de l'acide dichloracétique par réaction des esters correspondants de l'acide glyoxylique avec le pentachlorure de phosphore, caractérisé en ce qu'on fair réagir un ester de l'acide glyoxylique de formule générale $R_1$—CO—COOR$_2$, où $R_1$ représente un groupe phényle, naphtyle, pyridyle, furyle ou thiényle, lesdits groupes étant éventuellement substitués par un ou plusieurs substituants du groupe de Cl, $NO_2$, alkoxy avec 1—8 atomes de carbone, et alkyle avec 1—8 atomes de carbone, et $R_2$ représente un groupe alkyle, cycloalkyle ou aryle, avec le pentachlorure de phosphore, à une température de 35—150°C, en présence d'un dispersant convenant à la dispersion du pentachlorure de phosphore, ayant un point d'ébullition de 35—150°C, avec formation du dérivé dichloro correspondant, de formule générale $R_1$—CCl$_2$—COOR$_2$, où $R_1$ et $R_2$ ont la même signification que ci-dessus.

2. Procédé selon revendication 1, caractérisé en ce qu'on effectue la reaction à une température de 70—100°C.